# EUROPÄISCHE PATENTSCHRIFT

(11) **EP 1 184 144 B1**
(45) Veröffentlichungstag und Bekanntmachung des Hinweises auf die Patenterteilung: **11.11.2009**
(21) Anmeldenummer: 01120368.4
(22) Anmeldetag: 25.08.2001
(51) Int. Cl.: B27N 3/00

(54) **Verfahren zur enzymatischen Aktivierung von lignocellulosen Faserstoffen zur Herstellung von Werkstoffen**
Method for the enzymatic activation of lignocellulosic fibre materials for the manufacturing of products
Procédé pour l'activation enzymatique de matériaux en fibres à base de lignocellulose pour la fabrication de produits

(30) Priorität: 30.08.2000 DE 10043662
(43) Veröffentlichungstag der Anmeldung: 06.03.2002
(73) Patentinhaber: Sächsisches Institut für Angewandte Biotechnologie e.V. (SIAB), 04318 Leipzig (DE); Technische Universität Dresden, 01069 Dresden (DE)
(72) Erfinder: Unbehaun, Holger, Dipl.-Ing., 01099 Dresden (DE); Kerns, Gerhard, Dr., 04209 Leipzig (DE); König, Swetlana Dr., 04229 Leipzig (DE); Kühne, Gerhard, Prof. Dr., 01326 Dresden (DE); Wagenführ, André, Prof. Dr., 01109 Dresden (DE); Bley, Thomas, Prof. Dr., 04275 Leipzig (DE)

(56) Entgegenhaltungen:
- EP-A- 0 565 109
- US-A- 5 846 788

## Beschreibung

Die Erfindung betrifft ein Verfahren gemäß dem Oberbegriff des Hauptanspruches 1 zur Herstellung von lignocellulosen Faserwerkstoffen gemäß den Ansprüchen 20 bis 23.

Faserwerkstoffe in Form von MDF (Medium-Density-Fiber-Boards), HDF (High-Density-Fibre-Boards) oder Faserdämmplatten werden vielfältig in der Möbel- und Verpackungsindustrie, im Baugewerbe und im Automobilbau eingesetzt. Diese Werkstoffe werden im allgemeinen aus Faserstoff hergestellt, der durch thermo-mechanischen Aufschluss (Zerfaserung) im Defibrator oder Refiner aus Holzhackschnitzeln gewonnen wird. Der Faserstoff wird mit synthetischen Harzen, z.B. Harnstoff-, Phenol- bzw. Melaminformaldehydharz oder Diisocyanten beleimt, getrocknet und zu einem Vlies geformt. Der Bindemittelanteil beträgt üblicherweise 3 bis 20 Gew. % bezogen auf die Fasermasse (atro). Das Verpressen zu plattenförmigen Werkstoffen erfolgt in kontinuierlichen und diskontinuierlichen Verfahren. In Abhängigkeit von der Dichte werden Dämmplatten (ca. 200 bis 400 kg/m³), mittelharte Faserplatten (350 bis 800 kg/m³), MDF (650 bis 900 kg/m³) und HDF (800 bis 1200 kg/m³) produziert. Weiterhin werden Verfahren angewendet, bei denen die Fasern nach dem Streuen in einem ersten Pressvorgang vorgeformt werden und später mit mehrdimensional gestalteten Presswerkzeugen zu Faserformteilen verarbeitet werden. Zur Herstellung von Holzwerkstoffen in konventionellen Verfahren werden große Mengen synthetischer Klebstoffe und Bindemittel eingesetzt, die hohe Kosten verursachen sowie ökologische und gesundheitliche Risiken beinhalten.

Als Alternative zum Einsatz der genannten synthetischen Klebstoffe wurden eine Reihe von Verfahren entwickelt, bei denen Bindemittel auf biologischer Basis oder das stoffeigene Lignin der Faseroberfläche für Bindungen genutzt werden. So wird gemäß DE 30 37 992 A1 Ligninsulfonat der Sulfitablauge aus der Zellstoffproduktion mit phenoloxidierenden Enzymen versetzt, mit Holzpartikeln vermischt und zu Spanplatten verarbeitet. Die Enzyme bewirken dabei eine oxidative Polymerisation der phenolischen Komponente, die zu einer Verklebung der Holzpartikel führt. Hier wurde die Ligninkomponente in gelöster Form verarbeitet. Gemäß DE 43 05 411, DE 43 40 518, WO 94/01488 und WO 95/07604 wird das ungelöste Lignin an der Faseroberfläche direkt mit phenoloxidierenden Enzymen, wie z.B. Laccase oder Peroxidasen, behandelt. Dadurch werden Phenoxyradikale erzeugt und das Lignin der Faseroberfläche reaktiviert. Danach erfolgt eine Verarbeitung des Faserstoffes zu Platten mit verbesserten Faserbindungen. Ziel der Verfahren ist die Herstellung bindemittelfreier Formkörper. Die genannten Verfahren konzentrieren sich ausschließlich auf die enzymatische Aktivierung der Ligninkomponente und deren Nutzung für die Erhöhung der Werkstofffestigkeit. Aufgrund der bisher recht begrenzten kommerziellen Nutzung von Phenoloxidasen sind diese, beispielsweise gegenüber Xylanasen, relativ teuer. Die in den Quellen aufgeführten Inkubationszeiten betragen mehrere Stunden bis Tage und sind daher für eine breite Anwendung in hochproduktiven Verfahren wenig geeignet.

Hydrolasen werden gemäß WO 94/20672 A1 in sogenannten Biobleaching-Verfahren zur Bleiche von Zellstoff eingesetzt. Dabei werden die Fasern in Suspension mit Xylanasen und anderen Chemikalien zur Entfernung des Lignins behandelt. Die Zielstellung besteht hier in der Loslösung des Lignins von der Cellulose und nicht in einer Aktivierung von Komponenten der Lignocellulose mit dem Ziel der Verklebung, also einer Festigung des Verbundes aller Komponenten.

Es sind Arbeiten zum Einsatz von Hydrolasen bei der Herstellung von Werkstoffen bekannt, die sich auf die Gewinnung von Bindemitteln beschränken. So ist in DE 4340518 A1 der Einsatz von Hydrolasen zur Modifikation von Kartoffelpülpe beschrieben, die dann ihrerseits als Bindemittel eingesetzt wird; der Pülpeeinsatz erfordert dabei eine Trockenzeit von mehr als sieben Stunden. In WO 98/31762 ist ein Verfahren beschrieben, bei dem Holzpartikel mit Hydrolasen behandelt werden, um durch Teilhydrolyse Extraktstoffe (lösliche Bestandteile) zu gewinnen und danach unter Zugabe von Laccase ein Bindemittel herzustellen und dieses für die Verklebung von Fasern und Spänen zu nutzen. Auch hier ist eine schon erwähnte oxidative Aktivierung des Faserstoffes vorgesehen. In dem russischen Patent U.S.S.R 636311 wird der Einsatz von Hydrolasen und Oxidasen zur Plastifizierung von Hackschnitzeln und anderen Holzpartikeln vor der Zerfaserung beschrieben. Ziel ist hier eine Energieeinsparung beim Mahlen.

Die EP-A-0 565 109 offenbart ein Verfahren zum Verkleben von Holzfasern, deren Mittellamellen-Lignin durch phenoloxidierende Enzyme modifiziert wird.

Die Aufgabe der Erfindung besteht jedoch in der Bereitstellung eines Verfahrens zur Herstellung von lignocellulosen Faserwerkstoffen mit reduziertem oder völlig ohne Bindemittelanteil. Die Aufgabe der Erfindung wird gelöst durch ein Verfahren gemäß Anspruch 1. Die Unteransprüche 2 bis 19 betreffen bevorzugte Ausführungsvarianten des erfindungsgemäßen Verfahrens. Gemäß Anspruch 1 basiert das dieser Anmeldung zugrundeliegende Verfahren auf einer direkten Inkubation von Fasern während oder nach der Zerfaserung mit nachfolgender Weiterverarbeitung zu Werkstoffen. Die mit dem erfindungsgemäßen Verfahren erhältlichen Werkstoffe sind in den Ansprüchen 20 bis 23 genannt.

Als Ausgangsmaterial für das erfindungsgemäße Verfahren dienen Faserstoffe, vorzugsweise aus Holz, aber auch aus anderen lignocellulosen Stoffen wie Rapsstroh, Flachs, Hanf, Getreidestroh, Kokosfasern, Bambus, Reisstroh, Bagasse, u.a., die für Enzyme zugänglich sind. Die genannten Ausgangsmaterialien, z.B. Holzhackschnitzel, werden vorzugsweise auf konventionelle Weise im Defibrator nach einem thermomechanischen Verfahren bei Temperaturen von 160 bis 180 °C zerfasert. Andere Faserpflanzen wie Raps-, Flachs-, und Getreidestroh können auch ohne thermische Vorbehandlung rein mechanisch im Refiner oder im Extruder zerfasert werden. Die Separierung der Fasern kann auch, wie in der Zellstoffindustrie üblich, durch Kombination von thermischer Vorbehandlung, mechanischer Zerfaserung und chemischem Aufschluss nach Sulfit-, Sulfat- oder Organosolv- Verfahren erfolgen. Eine alternative Variante dazu ist das Dampfexplosionsverfahren nach Mason. Bei diesem Verfahren wird das Ausgangsmaterial nach Behandlung mit Dampf und Druck schnell entspannt und aufgrund des Berstdruckes werden die Fasern voneinander getrennt.

Die Fasern werden mit den Enzymen durch Sprühen, Tauchen oder Tränken benetzt. Die Art der Benetzung wird in Abhängigkeit von Weiterverarbeitungsverfahren und der Enzymkonzentration gewählt. Das Aufsprühen von hochkonzentrierten Enzymen erweist sich insbesondere bei der Werkstoffherstellung im Trockenverfahren als vorteilhaft, da die Vliesfeuchte vor dem Pressen vorzugsweise nicht höher als 25 Gew. % liegen sollte. Liegen die Enzyme trocken in Form von Pulver oder Pellets vor, können diese mit den Fasern auch im trockenen Zustand vermischt werden. Zur Verarbeitung der Fasern im Nass- und Halbtrockenverfahren können diese mit der Enzymlösung durch Tauchen oder Tränken benetzt werden, da hier höhere Feuchteanteile üblich sind.

Diese Aktivierung des Faserstoffes soll durch enzymatische Modifikation der Hemicellulose- und/oder Cellulosebestandteile mit Hilfe von Hydrolasen bzw. durch eine kombinierte Inkubation mit Oxidasen zur zusätzlichen Aktivierung der Ligninkomponente erreicht werden. Vorzugsweise sollen dabei Xylanasen bzw. Xylanasen in Kombination mit Phenoloxidasen, wie Laccase oder Peroxidase, zum Einsatz kommen. Gemäß dem Patentanspruch 6 und den Beispielen 1 und 2 kann auch eine Xylanase-/Cellulase-Enzymlösung, gewonnen aus einer Submerskultur von *Trichoderma reesei*, eingesetzt werden. Im Gegensatz zu bisher bekannten Methoden sollen bei der Ausbildung von Faser zu Faserbindungen nicht nur das Lignin, sondern vor allem Hemicellulose und Cellulose aktiviert werden. Insbesondere Xylanasen werden bereits in großtechnischem Maßstab hergestellt und sind preiswert zu beziehen. Die der Benetzung der Fasern folgende Inkubation sollte bei den für die Enzyme optimalen Temperaturen und pH-Werten stattfinden. Für Xylanase aus *T*. *reesei* liegen diese bei ca. 50°C und einem pH-Wert von 4,5 bis 5. Die Inkubationszeit kann in Abhängigkeit von den eingesetzten Enzymaktivitäten von wenigen Sekunden bis zu mehreren Tagen reichen. Durch thermische und chemische Denaturierung sowie durch Zugabe von Enzyminhibitoren oder durch Entzug der Enzyme kann die Reaktion abgebrochen werden.

Durch Einwirken von Enzymen an der Faseroberfläche werden stoffeigene Bindekräfte von Komponenten der Lignocellulose aktiviert, so dass sich bei einer Verpressung der Fasern zu Werkstoffen vermehrt Bindungen zwischen den Fasern ausbilden können. Durch Kombination von Hydrolasen mit Oxidasen können Synergieeffekte durch die kombinierte Aktivierung der verschiedenen Komponenten der Lignocellulose zur Verbesserung von Festigkeits- und hygroskopischen Eigenschaften genutzt werden. Die enzymhaltige Lösung kann auch andere Hilfsstoffe, Emulgatoren und Effektoren enthalten, die eine fördernde Funktion im Sinne der Erfindungsaufgabe erfüllen.

Nach der Inkubation lassen sich die Fasern im Nass-, Trocken- und Halbtrockenverfahren zu Werkstoffen verarbeiten. Beim Trockenverfahren müssen die Fasern auf eine Restfeuchte unter 25 Gew.- % getrocknet werden, bevor sie zu einem Vlies gestreut und verpresst werden. Sollten die durch eine enzymatische Aktivierung erreichten Eigenschaftsverbesserungen für spezielle Einsatzgebiete nicht ausreichend sein, können dem getrockneten Faserstoff o.g. konventionelle und alternative Bindemittel in reduzierter Menge zugesetzt werden. Bei dem Halbtrockenverfahren beträgt die erwähnte Restfeuchte 20 bis 35 Gew.- % und beim Nassverfahren bis zu 120 %. Auch hier können Bindemittel nach konventionellen Methoden zugemischt werden, falls das für die Erreichung der gewünschten Festigkeiten notwendig ist. Die Trocknung des Faserstoffes vor dem Verpressen kann in Strom- und Bandtrocknern bei Temperaturen zwischen 30 und 150°C erfolgen, vorzugsweise mit Temperaturen zwischen 40 und 90 °C. Der getrocknete Faserstoff wird zu Vliesen verarbeitet und unter Einfluss von Wärme und Druck verpresst. Das Heißpressen kann in Takt- oder kontinuierlichen Pressen nach üblichen Regimes erfolgen. Die hergestellten Werkstoffe aus enzymbehandeltem Faserstoff besitzen gegenüber Werkstoffen aus unbehandeltem Faserstoff erheblich bessere Quellungs- und mechanische Eigenschaften, wie Biege- und Querzugfestigkeiten. Die Dichte der hergestellten Werkstoffe kann im Bereich von 100 bis 1200 kg/m³ liegen, bei MDF vorzugsweise im Bereich von 700 bis 900 kg/m³ und bei Dämmplatten im Bereich von 150 bis 450 kg/m³.

Die Werkstoffe können vielfältig in der Möbel- und Bauindustrie, im Verpackungsbereich und im Automobilbau eingesetzt werden.

Die in der Erfindung beschriebenen Werkstoffe erfüllen aufgrund des reduzierten bzw. völlig substituierten Anteils an synthetischen Bindemitteln Anforderungen an eine umwelt- und gesundheitsschonende Herstellung, Nutzung und Entsorgung. Ein reduzierter Bindemittelanteil führt zu Einsparungen an Produktions- und Entsorgungskosten.

Es ist überraschend und nicht naheliegend, dass Hydrolasen, insbesondere Xylanasen, derart auf lignocellulose Komponenten einwirken, dass der Effekt einer Verklebung der Fasern untereinander eintritt und dieser Effekt bei der Herstellung von Holzwerkstoffen genutzt werden kann.

Die Erfindung wird im folgenden anhand der Beispiele näher erläutert.

### Beispiel 1

Konventioneller Holzfaserstoff wird mit einer Enzym- Pufferlösung inkubiert. Die Enzym-Pufferlösung besteht aus Citratpuffer mit einem pH-Wert von 4,5 und einer aufkonzentrierten Fermenterkultur von *Trichoderma reseei* mit folgender Zusammensetzung: Xylanaseaktivität 2500 IU/ml; Cellulaseaktivität 43 IU/ml Filterpapieraktivität und 3300 IU/ml CMC-Aktivität (Die Aktivitäten sind nach den Empfehlungen der IUPAC-Commission on Biotechnology durchgeführt. Die Methoden zur Xylanasebestimmung sind beschrieben in: Pure & Appl. Chem., Vol. 59, No. 12, pp. 1739-1752, 1987, die Methode zur Bestimmung der Filterpapieraktivität in: Pure & Appl. Chem., Vol. 59, No. 2, pp. 257-268, 1987). Die Verdünnung erfolgte 1:5. Die Inkubation erfolgte bei 50 °C über 10 min. Aus dem Faserstoff wurden nach dem Rapid-Köthen-Verfahren Faserblätter gebildet und deren Reißlänge bestimmt. Die Reißlänge stieg infolge der Inkubation von 0,14 km um 93 % auf 0,27 km.

### Beispiel 2:

Konventioneller Holzfaserstoff wurde mit 100 Gew. % (bezogen auf atro Fasermasse) Enzym-Pufferlösung besprüht wobei hier die Aktivitäten der eingesetzten Enzymlösung nur halb so hoch waren, wie im Beispiel 1. Die Inkubationszeit betrug eine Stunde bei 50 °C. Die Biegefestigkeit der hergestellten bindemittelfreien MDF mit einer Dichte von 800 kg/m³ betrug 10,79 N/mm². Die ohne Enzym hergestellte MDF hatte eine Biegefestigkeit von 7,1 N/mm². Die Festigkeit verbesserte sich somit um 52 %.

### Beispiel für die Herstellung der Enzymlösung

Der Pilz *Trichoderma reesei* M18.2, welcher aus der Mutante *T. reesei* ZIMET 43803 selektiert wurde, wird für die Xylanasefermentation nach den allgemein bekannten Fermentationstechniken unter den folgenden Bedingungen kultiviert:

Die Fermentation wird in einem Rührfermenter mit einem Bruttovolumen von 80 Litern unter Ausschluss einer Sauerstofflimitation durchgeführt. Das Anfangsvolumen beträgt 50 Liter mit folgender Zusammensetzung des Nährmediums:

| | |
|---|---|
| KH₂P0₄ | 3 g/l |
| (NH₄)₂S0₄ | 4,8 g/l |
| CaCl₂ | 0,4 g/l |
| MgSO₄ x 7 H2O | 0,4 g/l |
| Tween 80 | 2 g/l |
| Lactose | 15 g/l |
| Dünnschlempe | 50 Vol.-% |
| (z.B. Dünnschlempe aus der Brennerei Nordhausen) | |
| Cellulosepulver | 20 g/l |

Das Nährmedium wird mit 1,6 Liter einer Vorkultur des o.g. Stammes beimpft, welcher in Schüttelkolben auf Glucose als Kohlenstoffquelle angezogen wurde (z.B. 8 Kolben mit je 200 ml Arbeitsmedium).

Nach 48 Stunden Kultivierung werden 1,2 kg Cellulose in 5 Liter Leitungswasser plus 5 Liter

Dünnschlempe zugegeben.

Folgende Parameter werden für den Fermentationsprozess eingestellt:
- Anfangstemperatur 30°C, nach 36 Stunden wird die Temperatur auf 28°C abgesenkt
- Drehzahl geregelt, so dass der O₂-Partialdruck im Medium größer als 20 % ist
- pH: 5,5 (geregelt mit 12%-iger Ammoniaklösung)
- Begasung: 101/min Luft.

Nach 112 Stunden wird die Fermentation beendet. Die Xylanaseaktivität beträgt 550 IU/ml, die Cellulaseaktivität 7,5 FPU/ml. Die Aufarbeitung des Kulturmediums erfolgt nach den allgemein bekannten Verfahren (Abtrennung der Biomasse, Mikrofiltration und ggf. Ultrafiltration des Kulturfiltrates zur Aufkonzentrierung der Enzyme).

An Stelle des hier eingesetzten *T. reesei*-Stammes können auch andere *T. reesei*-Mutanten verwendet werden, die für die Xylanasebildung geeignet sind. Durch automatisch gesteuerte *fed-batch*-Techniken (z.B. *feedback*-kontrollierte*-fed-batch-*Technik, angesteuert über Abgas-CO₂) kann die Enzymbildung erhöht werden.

### Beispiel 3:

Konventioneller Holzfaserstoff wurde mit 100 Gew. % Enzym-Pufferlösung besprüht. Die Enzym-Pufferlösung bestand aus einem Puffer mit einem pH-Wert von 7 und einem

Enzympräparat mit hoher Cellulaseaktivität (38 000 NCU/g). Die Aktivität wurde an CMC als Enzymsubstrat bei 50 °C und pH 7 bestimmt.

Nach einer Inkubationszeit von 5 min wurde der Faserstoff in einem Stromtrockner auf etwa 11 % Holzfeuchte (bezogen auf atro Faserstoffgewicht) getrocknet. Die Vliesbildung erfolgte in einem Trockenverfahren ohne Zusatz von Bindemitteln. Unmittelbar nach dieser Vliesbildung wurde die trockene Fasermatte bei 200 °C zu einer 6 mm dicken MDF verpresst.

Zur Gewinnung von Vergleichswerten wurde statt der Enzym-Pufferlösung Wasser zur Benetzung der Fasern verwendet. Die Eigenschaften der hergestellten MDF sind in folgender Tabelle aufgeführt.

| **Meßgröße** | **Variante 1: Enzym-Pufferlösung** | **Variante 2: Kontrolle mit Wasser** |
|---|---|---|
| Dicke (mm) | 6,1 | 7,0 |
| Dichte (kg/m³) | 794 | 700 |
| Biegefestigkeit (N/mm²) | 36,46 | 8,18 |
| Elastizitätsmodul (N/mm²) | 4485 | 1528 |
| Querzugfestigkeit (N/mm²) | 0,64 | 0,06 |
| 24 h Quellung (%) | 25 | 81 |

Bei den Festigkeiten werden bereits Standartwerte erreicht. Die Quellwerte liegen leicht darüber. Durch Einsatz von geringen Mengen Hydrophobierungsmittel können auch bei den Quellwerten die Normen eingehalten werden.

## Patentansprüche

1. Verfahren zur Herstellung von lignocellulosen Faserwerkstoffen durch Zerfaserung von lignocellulosen Materialien und Weiterverarbeitung der Fasern zu Platten und Formkörpern oder anderen Werkstoffen, **dadurch gekennzeichnet, dass** die lignocellulosen Partikel während oder nach dem Zerfaserungsprozess und vor ihrer Weiterverarbeitung mit hydrolytischen Enzymen inkubiert und aktiviert werden.

2. Verfahren nach Anspruch 1, **dadurch gekennzeichnet, dass** es sich bei den lignocellulosen Materialien um Holz, Stroh, Flachs, Raps, Baumwollstroh, Kokosfasern, Bagasse, Reisstroh, Getreidestroh, Bambus oder anderen lignocellulosen Stoffen handelt.

3. Verfahren nach Anspruch 1 und 2, **dadurch gekennzeichnet, dass** die Fasern durch mechanischen, thermomechanischen oder chemischen Aufschluss oder deren Kombination gewonnen werden.

4. Verfahren nach mindestens einem der Ansprüche 1 bis 3, **dadurch gekennzeichnet, dass** die Fasern durch Dampfexplosionsverfahren hergestellt werden.

5. Verfahren nach mindestens einem der Ansprüche 1 bis 4, **dadurch gekennzeichnet, dass** als Enzyme Hydrolasen eingesetzt werden.

6. Verfahren nach mindestens einem der Ansprüche 1 bis 5, **dadurch gekennzeichnet, dass** als Enzyme Cellulasen und/oder Hemicellulasen eingesetzt werden.

7. Verfahren nach mindestens einem der Ansprüche 1 bis 6, **dadurch gekennzeichnet, dass** Hydrolasen in Kombination mit Oxidasen eingesetzt werden.

8. Verfahren nach Anspruch 1 bis 7, **dadurch gekennzeichnet, dass** die Enzyme in Wasser oder Puffer mit einem pH-Wert im Bereich von 3 bis 9 gelöst werden.

9. Verfahren nach Anspruch 1 bis 8, **dadurch gekennzeichnet, dass** die Inkubation der Fasern in einem Temperaturbereich von 15 bis 90 °C und bei einem pH-Wert im Bereich von 3 bis 9 erfolgt.

10. Verfahren nach mindestens einem der Ansprüche 1 bis 9, **dadurch gekennzeichnet, dass** die Lösung der Enzyme Hilfsstoffe wie Emulgatoren, Effektoren und/oder andere Enzyme mit unterstützender Funktion enthält.

11. Verfahren nach mindestens einem der Ansprüche 1 bis 10, **dadurch gekennzeichnet, dass** die Einwirkzeit der Enzyme 10 Sekunden bis 30 Tage betragen kann.

12. Verfahren nach mindestens einem der Ansprüche 1 bis 11, **dadurch gekennzeichnet, dass** die Enzyme in gelöster Form durch Sprühen, Gießen, Tauchen und/oder Trocken mit den lignocellulosen Faserpartikeln in Kontakt gebracht werden.

13. Verfahren nach mindestens einem der Ansprüche 1 bis 12, **dadurch gekennzeichnet, dass** die Inkubation bei einer Faserstofffeuchte von mind. 8 % stattfindet.

14. Verfahren nach mindestens einem der Ansprüche 1 bis 13, **dadurch gekennzeichnet, dass** die Inkubation der Fasern in Suspension mit dem Enzym erfolgt.

15. Verfahren nach mindestens einem der Ansprüche 1 bis 14, **dadurch gekennzeichnet, dass** die Inkubation durch Zugabe von Enzyminhibitoren, durch Denaturierung der Enzyme oder durch Entzug der Enzymlösung beendet wird.

16. Verfahren nach mindestens einem der Ansprüche 1 bis 15, **dadurch gekennzeichnet, dass** nach der Inkubation ein teilweiser Wasserentzug erfolgt, der im Trockenverfahren bis zu einer Restfeuchte von weniger als 20 Gew.-%, im Halbtrockenverfahren bis zu einer Restfeuchte von 20 bis 35 Gew.-% und im Nassverfahren bis zu einer Restfeuchte von 120 Gew.-% durchgeführt wird.

17. Verfahren nach mindestens einem der Ansprüche 1 bis 16, **dadurch gekennzeichnet, dass** die Herstellung von Formkörpern neben mechanischen wirkenden Verfahren auch durch thermische wirkende Verfahren erfolgt.

18. Verfahren nach mindestens einem der Ansprüche 1 bis 17, **dadurch gekennzeichnet, dass** der vorbereitete Faserstoff zu einem vliesartigen Vorprodukt geformt, vorverdichtet, in die gewünschte Form gebracht und danach unter Druck und gegebenenfalls Hitzeeinwirkung verpresst wird ohne und mit Anwesenheit von konventionellen Bindemitteln.

19. Verfahren gemäss einem der Ansprüche 1 bin 18, **dadurch gekennzeichnet, dass** die Verpressung zu Faserwerkstoffen ein- oder mehrstufig erfolgt und unter weiterer Verdichtung und gegebenenfalls Formgebung zu Faserwerkstoffen.

## Claims

1. Process for the production of lignocellulosic fibre materials by defibration of lignocellulosic materials and further processing of the fibres to give sheets and mouldings or other materials, **characterized in that** the lignocellulosic particles are incubated and activated with hydrolytic enzymes during or after the defibration process and before their further processing.

2. Process according to Claim 1, **characterized in that** the lignocellulosic materials are wood, straw, flax, rape, cotton straw, coconut fibres, bagasse, rice straw, cereal straw, bamboo or other lignocellulosic substances.

3. Process according to Claims 1 and 2, **characterized in that** the fibres are obtained by mechanical, thermomechanical or chemical digestion or a combination thereof.

4. Process according to at least one of Claims 1 to 3, **characterized in that** the fibres are produced by steam explosion processes.

5. Process according to at least one of Claims 1 to 4, **characterized in that** the enzymes used are hydrolases.

6. Process according to at least one of Claims 1 to 5, **characterized in that** the enzymes used are cellulases and/or hemicellulases.

7. Process according to at least one of Claims 1 to 6, **characterized in that** hydrolases are used in combination with oxidases.

8. Process according to Claims 1 to 7, **characterized in that** the enzymes are dissolved in water or buffer having a pH in the range from 3 to 9.

9. Process according to Claims 1 to 8, **characterized in that** the incubation of the fibres is effected in a temperature range from 15 to 90°C and at a pH in the range from 3 to 9.

10. Process according to at least one of Claims 1 to 9, **characterized in that** the solution of the enzymes contains auxiliaries, such as emulsifiers, effectors and/or other enzymes having a supporting function.

11. Process according to at least one of Claims 1 to 10, **characterized in that** the action time of the enzymes may be 10 seconds to 30 days.

12. Process according to at least one of Claims 1 to 11, **characterized in that** the enzymes are brought into contact in dissolved form, by spraying, pouring, immersion, and/or in dry form with the lignocellulosic fibre particles.

13. Process according to at least one of Claims 1 to 12, **characterized in that** the incubation takes place at a fibre moisture content of at least 8%.

14. Process according to at least one of Claims 1 to 13, **characterized in that** the incubation of the fibres is effected in suspension with the enzyme.

15. Process according to at least one of Claims 1 to 14, **characterized in that** the incubation is terminated by adding enzyme inhibitors, by denaturing the enzymes or by removing the enzyme solution.

16. Process according to at least one of Claims 1 to 15, **characterized in that**, after incubation, partial removal of water is effected, which is carried out to a residual moisture content of less than 20% by weight in the dry process, to a residual moisture content of 20 to 35% by weight in the semidry process and to a residual moisture content of 120% by weight in the wet process.

17. Process according to at least one of Claims 1 to 16, **characterized in that** the production of the mouldings is also effected by thermal processes in addition to mechanical processes.

18. Process according to at least one of Claims 1 to 17, **characterized in that** the prepared fibre material is formed into a nonwoven-type precursor, precompacted, brought into desired shape and then pressed under pressure and optionally under the action of heat, in the absence of presence of conventional binders.

19. Process according to any of Claims 1 to 18, **characterized in that** the pressing to give fibre materials is effected in one or more stages and with further compaction and optionally shaping to give fibre materials.

## Revendications

1. Procédé de fabrication de matériaux fibreux à base de lignocellulose par défibrage de matériaux lignocellulosiques et transformation des fibres en plaques, en corps façonnés ou en d'autres matériaux,
**caractérisé en ce que**
les particules lignocellulosiques sont incubées avec des enzymes hydrolytiques et activées pendant ou après l'opération de défibrage et avant la poursuite de leur traitement.

2. Procédé selon la revendication 1, **caractérisé en ce que** les matériaux lignocellulosiques sont le bois, la paille, le lin, le colza, la bourre de coton, les fibres de noix de coco, la bagasse, la paille de riz, la paille de céréales, le bambou ou d'autres substances lignocellulosiques.

3. Procédé selon les revendications 1 et 2, **caractérisé en ce que** les fibres sont obtenues par désagrégation mécanique, thermomécanique ou chimique ou combinaisons de ces opérations.

4. Procédé selon au moins l'une des revendications 1 à 3, **caractérisé en ce que** les fibres sont préparées par un procédé d'éclatement à la vapeur.

5. Procédé selon au moins l'une des revendications 1 à 4, **caractérisé en ce que** les enzymes utilisées sont des hydrolases.

6. Procédé selon au moins l'une des revendications 1 à 5, **caractérisé en ce que** les enzymes utilisées sont des cellulases et/ou hémicellulases.

7. Procédé selon au moins l'une des revendications 1 à 6, **caractérisé en ce qu'**il utilise des hydrolases combinées avec des oxydases.

8. Procédé selon les revendications 1 à 7, **caractérisé en ce que** les enzymes sont dissoutes dans l'eau ou dans un tampon dont la valeur du pH est comprise dans la plage de 3 à 9.

9. Procédé selon les revendications 1 à 8, **caractérisé en ce que** l'incubation des fibres a lieu dans une plage de température de 15 à 90°C et à une valeur du pH comprise dans la plage de 3 à 9.

10. Procédé selon au moins l'une des revendications 1 à 9, **caractérisé en ce que** la solution d'enzymes contient des substances auxiliaires, par exemple des émulsifiants, des effecteurs et/ou d'autres enzymes à fonction d'assistance.

11. Procédé selon au moins l'une des revendications 1 à 10, **caractérisé en ce que** la durée d'action des enzymes peut s'étendre entre 10 secondes et 30 jours.

12. Procédé selon au moins l'une des revendications 1 à 11, **caractérisé en ce que** les enzymes sont mises en contact avec les particules de fibres lignocellulosiques sous forme soluble, par pulvérisation, versage, immersion et/ou séchage.

13. Procédé selon au moins l'une des revendications 1 à 12, **caractérisé en ce que** l'incubation a lieu à une humidité des fibres d'au moins 8 %.

14. Procédé selon au moins l'une des revendications 1 à 13, **caractérisé en ce que** l'incubation des fibres s'effectue en suspension avec l'enzyme.

15. Procédé selon au moins l'une des revendications 1 à 14, **caractérisé en ce que** l'incubation est arrêtée par addition d'inhibiteurs d'enzymes, par dénaturation des enzymes ou par extraction de la solution d'enzyme.

16. Procédé selon au moins l'une des revendications 1 à 15, **caractérisé en ce qu'**après l'incubation, une partie de l'eau est extraite de telle sorte que dans un procédé à sec, l'humidité résiduelle soit inférieure à 20 % en poids, que dans un procédé semi-sec l'humidité résiduelle soit de 20 à 35 % en poids et que dans un procédé humide l'humidité résiduelle soit de 120 % en poids.

17. Procédé selon l'une des revendications 1 à 16, **caractérisé en ce que** la préparation de corps façonnés s'effectue par des procédés à action mécanique et/ou également par des procédés à action thermique.

18. Procédé selon au moins l'une des revendications 1 à 17, **caractérisé en ce que** la matière fibreuse préparée est façonnée en pré-produit de type feutre, est pré-compactée, est amenée dans la forme souhaitée et est ensuite comprimée sous pression et éventuellement action de la chaleur, en présence ou en absence de liants classiques.

19. Procédé selon l'une des revendications 1 à 18, **caractérisé en ce que** la compression en matériaux fibreux s'effectue en une ou plusieurs étapes et **en ce que** les matériaux fibreux sont obtenus par nouvelle compression et éventuellement nouveau façonnage.
